# EUROPEAN PATENT SPECIFICATION

(11) **EP 2 875 817 B1**
(45) Date of publication and mention of the grant of the patent: **18.03.2020**
(21) Application number: 13194485.2
(22) Date of filing: 26.11.2013
(51) Int. Cl.: A61K 31/454, A61K 9/16

(54) **Pharmaceutical formulation comprising amorphous lenalidomide**
Pharmazeutische Formulierung enthaltend amorphes Lenalidomid
Formulation pharmaceutique comprenant du lénalidomide amorphe

(43) Date of publication of application: 27.05.2015
(73) Proprietor: Synthon B.V., 6545 CM Nijmegen (NL)
(72) Inventor: Van Eupen, Jacobus Theodorus Henricus, 6545CM Nijmegen (NL); Keltjens, Rolf, 6545CM Nijmegen (NL); Murpani, Deepak, 6545CM Nijmegen (NL)
(74) Representative: Tejedor Vinent, Henar

(56) References cited:
- WO-A1-2010/054833
- WO-A2-2005/023192
- WO-A2-2009/114601
- US-A1- 2007 215 511

## Description

Lenalidomide is a pharmaceutically active compound used for the treatment of multiple myeloma. It is marketed under the name Revlimid by Celgene in the form of capsules that should be kept at a temperature below 30°C. The compound was disclosed in WO9803502. Lenalidomide exists in various solid forms. Various anhydrates and solvates are disclosed in WO2005023192. These forms can interconvert, e.g. hemihydrate B converts into dihydrate E in the presence of water, anhydrate A converts into form E in the presence of an amount of form E or in the presence of water. Furthermore, these forms differ in water solubility, which for all forms is relatively low.

The water solubility of amorphous forms is known to be higher compared to the water solubility of crystalline forms. In view of this, it would be desirable to stabilize lenalidomide in anamorphous form.

WO2010054833 discloses a solid solution of lenalidomide in a matrix material. These solid solutions are prepared using melt extrusion and spray drying. These pharmaceutical techniques have disadvantages: melt extrusion requires high temperatures. Since lenalidomide has a high melting point, this may result in growing of impurities as a result of thermal decomposition. Also, the extrudates require milling in order to be useful for preparing pharmaceutical formulations thereof. Spray drying is disadvantageous, because in solvents that are suitable for spray drying, lenalidomide has low solubility. Therefore, spray drying requires copious amounts of solvents, making the process unsuitable for carrying out on a commercial scale.

WO2009114601 discloses dispersions of lenalidomide in povidone. During the process to prepare these dispersions, a mixture of dimethylformamide and methanol is used. Spray drying/evaporation at high temperatures is necessary to completely remove these solvents. Using methanol and dimethylformamide is undesirable, as these solvents are toxic and are undesirable for making a pharmaceutical product.

Thus, in view of the prior art cited above there is still a need for compositions comprising lenalidomide that do not have the problems and disadvantages mentioned above.

### BRIEF DESCRIPTION OF THE INVENTION

The present invention provides a pharmaceutical composition comprising a solid composite consisting essentially of amorphous lenalidomide-ion exchange polymer with sulfonic acid groups complex. The invention further provides processes to prepare said formulations comprising the steps of intimately mixing lenalidomide with an ion exchange polymer in the H⁺ form in a liquid medium comprising water, or intimately mixing lenalidomide with an ion exchange polymer in a salt form in a liquid medium comprising water in the presence of an excess of an acid with a pKa of 1 or lower.

### DETAILED DESCRIPTION OF THE INVENTION

The present invention provides a pharmaceutical composition comprising a solid composite consisting essentially of amorphous lenalidomide ion exchange polymer with sulfonic acid complex, suitable polymers may be a sulfonated polystyrene or sulfonated copolymer of styrene and divinylbenzene or poly(2-acrylamido-2-methylpropane sulfonic acid). Most preferably, the pharmaceutical composition comprises a solid composite consisting essentially of lenalidomide and a sulfonated copolymer of styrene and divinyl benzene. Consisting essentially should be considered to mean that traces of other compounds may be present in the solid composite, e.g. residual salts or solvents. The weight ratio of lenalidomide to copolymer in the solid composite is at least 1:1.5, preferably from 1:1.5 to 1:10, more preferably from 1:2 to 1:4, most preferred is a ratio of 1:2 to 1:3.

In the pharmaceutical compositions of the present invention, lenalidomide is present in a stabilized amorphous form, which means that during stability studies no conversion of amorphous lenalidomide into any crystalline form was observed.

Preferably the composition according to the present invention is in the form of a tablet, a capsule or a sachet. In one embodiment of the invention, the pharmaceutical composition is a capsule or sachet filled with the solid composite consisting of lenalidomide and a sulfonated copolymer of styrene and divinylbenzene in the absence of additional excipient.

In an alternative embodiment of the invention, the pharmaceutical composition may additionally comprise one or more pharmaceutically acceptable excipients. The pharmaceutically acceptable excipients are chosen from one or more lubricants, fillers, binders, disintegrants or glidants. Suitable lubricants may be stearic acid, magnesium stearate, glyceryl behenate, glyceryl monostearate and palmitic acid. A preferred lubricant is magnesium stearate.

Suitable fillers may be polysaccharides, mono-or disaccharides, sugar alcohols, anorganic fillers, e.g. calcium silicate, dibasic calcium phosphate. Preferably lactose, microcrystalline cellulose or a mixture thereof is used as filler.

Suitable binders may be polysaccharides, mono-or disaccharides, modified celluloses, e.g. hydroxypropyl cellulose, sugar alcohols, synthetic polymers, e.g. polyvinylpyrrolidone, polyethylene glycol.

Suitable disintegrants may be crosscarmellose, crospovidone and sodium starch glycolate.

Suitable glidants may be colloidal silicon dioxide, powdered cellulose, hydrophobic colloidl silica, magnesium silicate, magnesium trisilicate, sodium stearate and talc.

The pharmaceutical formulations of the present invention display dissolution behavior typical for immediate release formulations. Immediate release typically means that 75% of the API is dissolved within 45 minutes, rapidly dissolving means that 85% is dissolved within 30 minutes. Very rapidly dissolving means that 85% is dissolved within 15 minutes. During preparation and storage of the pharmaceutical formulations of then present invention, the lenalidomide remains in amorphous form.

The invention further provides a solid composite consisting essentially of lenalidomide ion exchange polymer complex, preferably the ion exchange polymer has sulfonic acid groups, suitable polymers may be a sulfonated polystyrene or sulfonated copolymer of styrene and divinylbenzene or poly(2-acrylamido-2-methylpropane sulfonic acid). Consisting essentially should be considered to mean that traces of other compounds may be present in the solid composite, e.g. residual salts or solvents. Preferably the invention relates to a solid composite consisting essentially of lenalidomide and a sulfonated copolymer of styrene and divinyl benzene.

The weight ratio of lenalidomide to copolymer in the solid composite is at least 1:1.5, preferably from 1:1.5 to 1:10, more preferably from 1:2 to 1:4, most preferred is a ratio of 1:2 to 1:3.

The ion exchange polymer to be used in accordance with the present invention has sulfonic acid groups, the sulfonic acid groups are in the H⁺ form in the composite. Commercially available resins have sodium sulfonate groups, viz. are in the Na⁺ form, e.g. Amberlite IRP 69. The resin in the H+ form can be obtained by suspending the commercial available resin in water and adding a mineral acid solution, e.g. hydrochloric acid, filtering of the resin in the H⁺ form, and optionally washing off with water any excess of mineral acid and/or salts.

In the solid composite of the present invention, lenalidomide is present in a stabilized amorphous form, which means that during stability studies no conversion of amorphous lenalidomide into any crystalline form was observed. The solid composite advantageously is in the form of a free flowing powder, with excellent handling properties.

The solid composite is suitable to be used to prepare the pharmaceutical composition of the invention.

The invention further provides processes to prepare a pharmaceutical composition comprising a solid composite consisting essentially of amorphous lenalidomide ion exchange polymer with sulfonic acid groups complex comprising a stepI) of intimately mixing lenalidomide with an ion exchange polymer in the H+ form in a liquid medium comprising water, or intimately mixing lenalidomide with a ion exchange polymer in the salt form in a liquid medium comprising water in the presence of an acid with a pKa of 1 or lower, preferably an excess of a mineral acid. The most preferred acid is hydrochloric acid. The liquid medium comprising water is preferably water, or a mixture of water and an organic solvent. Preferably, the organic solvent is a polar solvent, preferred polar solvents are alcohols, particularly ethanol or methanol, ethers, particularly tetrahydrofuran, ketones, particularly acetone, and acetonitrile. If the process is performed in pure water, the composite may readily be isolated using filtration. As starting material for the process, all polymorphic forms of lenalidomide may be used, if the liquid medium is water, then form A as starting material is preferred. Form A is defined in WO2005023192 as a polymorph characterized by an XRPD pattern having peaks at approximately 8,14. 5,16, 17.5, 20.5, 24, and 26 degrees 2θ. As starting material for the process, the ion exchange polymer may be in several forms, either in the H+ form or in a salt form, e.g. Na+ form. If said copolymer is in the salt form, an excess of an acid with a pKa of 1 or lower should be present in the liquid medium comprising the ion exchange polymer and lenalidomide during said process. Preferably the acid used is a mineral acid, most preferably the acid is hydrogen chloride.

The process for preparing said pharmaceutical composition may additionally comprise the step II) of isolation of said solid composite using equipment and methods well-known in the art, followed by step III) of blending or granulating the composite with one or more pharmaceutically acceptable excipients. Alternatively, the suspension or solution obtained by step I) may directly, without isolation step II) be added to one or more pharmaceutically acceptable excipients in a wet granulation process. The obtained wet granules may subsequently be dried using equipment and methods well-known in the art. Preferably the process for preparing the pharmaceutical composition comprises additionally the step of compressing the resulting blend or granulates into tablets, or filling said blend or granulate into capsules or sachets. Alternatively, the process additionally comprises the step of directly filling capsules or sachets with the solid composite consisting of a lenalidomide ion exchange polymer complex in the absence of one or more additional excipients.

In an additional embodiment, the invention relates to a pharmaceutical composition obtainable by a process comprising the steps of I) intimately mixing lenalidomide with a sulfonated ion exchange polymer in the H+ form in a liquid medium comprising water, or intimately mixing lenalidomide with a sulfonated ion exchange polymer in a salt form in a liquid medium comprising water in the presence of an excess of a mineral acid, II) isolating a composite essentially consisting of an ion exchange complex of lenalidomide with a sulfonated ion exchange polymer III) optionally mixing with one or more pharmaceutically acceptable excipients, IV) compressing the resulting mixture into a tablet or filling the mixture into a capsule or sachet. Preferably the mineral acid is hydrochloric acid.

The solid composite and pharmaceutical formulation in accordance with the present invention may be used as medicament, preferably for the treatment of multiple myeloma. The present invention is illustrated by the following examples.

### Examples

### Example 1

### LNL-Amberlite IRP-69 1:3

To 3 g lenalidomide dissolved in 59 ml of water and 59 ml of ethanol while heating, was added 9 g of Amberlite IRP 69 (H⁺ form). The formed suspension was stirred at 60 °C for 2 hours. After cooling to room temperature, the solvent was evaporated and the residue was dried at 40°C under vacuum over night yielding 10.1 g solid composite. XRPD analysis of the solid showed absence of crystalline peaks after 3 months at 40 °C/75 % RH in an open dish.

### Example 2

### LNL-Amberlite IRP-69 1:3

0.33 g lenalidomide was added to a suspension of 1 g of Amberlite IRP
69 (H⁺ form) in 10 ml of water. The suspension was stirred at 60 °C for 3 hours.

After cooling to room temperature the solid was filtered off.

Yield after drying over night at 40°C under vacuum: 0.98 g. XRPD analysis of the solid showed absence of crystalline peaks.

### Example 3

### LNL-Amberlite IRP-69 1:2

Example 2 was repeated using lenalidomide and Amberlite IRP-69 in a weight ratio 1:2. XRPD analysis of the resulting composite showed absence of crystalline.

### Example 4

### LNL amberlite IRP 69 (Na⁺ form) complex

3 g LNL was dissolved in 40 ml 0.5 M HCl while heating. The solution was added to 9 g of Amberlite IRP 69 (Na+ form) suspended in 36 ml of water. The formed suspension was stirred at 60 °C for 2 hours. After cooling to room temperature the solvent was evaporated and the residue was dried at 40 °C under vacuum over night.

XRPD showed absence of crystalline peaks.

The material was put on stability in aluminum bags and remained amorphous after 2 months at 40°C /75 %RH.

### Example 5

Hard shell capsules were filled by hand with the composite of example 2. Dissolution was measured at different pH values using various media. In all conditions more than 80% of the lenalidomide is dissolved within 10 minutes, which is dissolution behavior typical for immediate release formulations.

## Claims

1. A pharmaceutical composition comprising a solid composite consisting essentially of amorphous lenalidomide ion exchange polymer with sulfonic acid groups complex.

2. The pharmaceutical composition according to claim 1, where the ion exchange polymer is a sulfonated polystyrene or a sulfonated copolymer of styrene and divinylbenzene or poly(2-acrylamido-2-methylpropane sulfonic acid).

3. The pharmaceutical composition according to any of the preceding claims, wherein the weight ratio of lenalidomide to sulfonated ion exchange polymer in the solid composite is at least 1:1.5.

4. The pharmaceutical composition according to claim 3 wherein said ratio is in the range of 1 to 1.5 to 1:10.

5. The pharmaceutical composition according to any of the preceding claims additionally comprising one or more pharmaceutically acceptable excipients.

6. The pharmaceutical composition according to any of the preceding claims in the form of a capsule.

7. A process for preparing a pharmaceutical composition comprising a solid composite consisting essentially of amorphous lenalidomide-ion-exchange polymer with sulfonic acid groups complex comprising the steps of
a) intimately mixing lenalidomide with a ion-exchange polymer in the H+ form in a liquid medium comprising water, or intimately mixing lenalidomide with a resin in the salt form in a liquid medium comprising water in the presence of an excess of an acid with a pKa of 1 or lower.
b1) isolating of said solid composite
c1) blending the composite with one or more pharmaceutically excipients
or
b2) isolating the solid composite
c2) granulating the composite with one or more pharmaceutically acceptable excipients.

8. The process according to claim 7, wherein lenalidomide form A with an XRPD pattern having peaks at approximately 8,14. 5,16, 17.5, 20.5, 24, and 26 degrees 2θ is used as starting material.

## Patentansprüche

1. Pharmazeutische Zusammensetzung, umfassend einen festen Verbundwerkstoff, der im Wesentlichen aus amorphem Lenalidomid-Ionenaustauschpolymer mit Sulfonsäuregruppenkomplex besteht.

2. Pharmazeutische Zusammensetzung nach Anspruch 1, wobei das Ionenaustauschpolymer ein sulfoniertes Polystyrol oder ein sulfoniertes Copolymer aus Styrol und Divinylbenzol oder Poly(2-acrylamido-2-methylpropansulfonsäure) ist.

3. Pharmazeutische Zusammensetzung nach einem der vorhergehenden Ansprüche, wobei das Gewichtsverhältnis von Lenalidomid zu sulfoniertem Ionenaustauschpolymer in dem festen Verbundwerkstoff mindestens 1:1.5 beträgt.

4. Pharmazeutische Zusammensetzung nach Anspruch 3, wobei das Verhältnis im Bereich von 1 zu 1.5 bis 1:10 liegt.

5. Pharmazeutische Zusammensetzung nach einem der vorhergehenden Ansprüche, zusätzlich umfassend einen oder mehrere pharmazeutisch annehmbare Trägerstoffe.

6. Pharmazeutische Zusammensetzung nach einem der vorhergehenden Ansprüche in Form einer Kapsel.

7. Verfahren zur Herstellung einer pharmazeutischen Zusammensetzung, die einen festen Verbundwerkstoff umfasst, der im Wesentlichen aus amorphem Lenalidomid-Ionenaustauschpolymer mit Sulfonsäuregruppenkomplex besteht, umfassend die folgenden Schritte
a) inniges Vermischen von Lenalidomid mit einem Ionenaustauschpolymer in H+-Form in einem flüssigen Medium, das Wasser umfasst, oder inniges Vermischen von Lenalidomid mit einem Harz in Salzform in einem flüssigen Medium, das Wasser umfasst, in Gegenwart eines Überschusses an Säure mit einem pKa-Wert von 1 oder niedriger.
b1) Isolieren des festen Verbundwerkstoffes
c1) Mischen des Verbundwerkstoffes mit einem oder mehreren pharmazeutischen Trägerstoffen
oder
b2) Isolieren des festen Verbundwerkstoffes
c2) Granulieren des Verbundwerkstoffes mit einem oder mehreren pharmazeutisch annehmbaren Trägerstoffen.

8. Verfahren nach Anspruch 7, wobei Lenalidomid der Form A mit einem XRPD-Muster mit Spitzen bei etwa 8,14. 5,16, 17.5, 20.5, 24 und 26 Grad 2θ als Ausgangsmaterial verwendet wird.

## Revendications

1. Composition pharmaceutique comprenant un composite solide constitué essentiellement d'un polymère d'échange d'ions de lénalidomide amorphe avec des groupes d'acide sulfonique.

2. Composition pharmaceutique selon la revendication 1, où le polymère d'échange d'ions est un polystyrène sulfoné ou un copolymère sulfoné de styrène et de diphénbenzène ou poly(acide sulfonique de 2-acrylamido-2-méthylpropane).

3. Composition pharmaceutique selon l'une quelconque des revendications précédentes, dans laquelle le rapport poids du lénalidomide au polymère d'échange d'ions sulfoné dans le composite solide est au moins 1:1.5.

4. Composition pharmaceutique selon la revendication 3, dans laquelle ledit rapport se situe dans la plage de 1 à 1.5 à 1:10.

5. Composition pharmaceutique selon l'une quelconque des revendications précédentes, comprenant en outre un ou plusieurs excipients pharmaceutiquement acceptables.

6. Composition pharmaceutique selon l'une quelconque des revendications précédentes en forme de gélule.

7. Procédé de préparation d'une composition pharmaceutique comprenant un composite solide constitué essentiellement d'un polymère d'échange d'ions amorphe de lénalidomide, avec un complexe de groupes d'acide sulfonique, comprenant les étapes consistant à a) mélanger intimement le lénalidomide avec un polymère d'échange d'ions dans la forme H+ dans un milieu liquide comprenant de l'eau, ou mélanger intimement le lénalidomide avec une résine en forme de sel dans un milieu liquide comprenant de l'eau en présence d'un excès d'acide avec un pKa de 1 ou moins.
b1) isoler ledit composite solide
c1) mélanger le composite avec un ou plusieurs excipients pharmaceutiquement acceptables
ou
b2) isoler le composite solide
c2) granuler le composite avec un ou plusieurs excipients pharmaceutiquement acceptables.

8. Procédé selon la revendication 7, dans lequel le lénalidomide de forme A avec un schéma XRPD ayant des pics à environ 8,14. 5,16, 17.5, 20.5, 24 et 26 degrés 2θ est utilisé comme matière de départ.
